# EUROPEAN PATENT APPLICATION

(11) **EP 4 442 684 A1**
(43) Date of publication of application: **09.10.2024**
(21) Application number: 22900625.9
(22) Date of filing: 01.12.2022
(51) Int. Cl.: C07D 401/14, A61K 31/506, A61P 35/00

(54) **A CLASS OF PYRIMIDINE COMPOUNDS, AND PREPARATION METHOD THEREFOR AND USE THEREOF**

(30) Priority: 01.12.2021 CN 202111452507
(71) Applicant: Shanghai Allist Pharmaceuticals Co., Ltd., Shanghai 201318 (CN)
(72) Inventor: ZHANG, Qiang, Shanghai 201318 (CN); WANG, Jiegang, Shanghai 201318 (CN); YE, Pinguo, Shanghai 201318 (CN)
(74) Representative: J A Kemp LLP
(86) International application number: PCT/CN2022/135922
(87) International publication number: WO 2023/098821

(57) **Abstract**

Provided are a pyrimidine compound, and a preparation method therefor and the use thereof. Specifically, provided is a compound as represented by formula I or a pharmaceutically acceptable salt thereof. The compound has a new structure, has a good inhibitory effect on EGFRDel19/T790M/C797S mutation, and is expected to treat and/or prevent a variety of diseases mediated by EGFR.

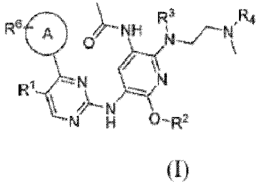

## Description

The present application claims priority to Chinese patent application 2021114525077 filed on December 1, 2021. The present application cites the above-mentioned Chinese patent application in its entirety.

### Technical Field

The present invention relates to a class of pyrimidine compounds, and a preparation method therefor and a use thereof.

### Background Art

Tumor is one of the most important problems that endanger human health, while lung cancer is one of the malignant tumors posing the greatest threat to people's health and life. Lung cancers are mainly divided into small cell lung cancer (SCLC) and non-small cell lung cancer (NSCLC), and among them, NSCLC accounts for about 80%. In non-small cell lung cancer, the most common mutation for which there are targeted drugs is "epidermal growth factor receptor" (EGFR) mutation. Therefore, use of an epidermal growth factor receptor EGFR inhibitor (an EGFR-TKI targeted drug) is the largest research hotspot in the treatment of lung cancer.

The epidermal growth factor receptor (EGFR) belongs to the protein tyrosine kinase (PTK) family, consisting of EGFR (Erb-B1), ErbB2 (HER-2/neu), Erb-B3 and Erb-B4, and is deemed as a crucial driving factor in the process of cell growth and proliferation. Overexpression and mutation of EGFR have been expressly proven to cause uncontrollable cell growth and are related to the course of most cancers, such as lung cancer, colon cancer, and breast cancer.

Currently, there are the first, second and third generations of marketed EGFR inhibitors. The first generation is reversible targeted drugs, for example, gefitinib, erlotinib, and icotinib. The second generation is irreversible targeted drugs, for example, afatinib and dacomitinib. Although the first- and second-generation targeted drugs have significant therapeutic effects, most patients will develop drug resistance after using the drugs for 1-2 years. Among the patients with resistance to EGFR inhibitors, 50% of the drug resistance is related to T790M mutation. The third-generation EGFR targeted drug (osimertinib) can bind to EGFR T790M mutation sites and inhibit tumor resistance caused by the T790M mutation, and its advent has brought good survival benefits to more patients with lung cancer. However, resistance to the third-generation targeted drugs also inevitably occurs due to the C797S mutation. The C797S mutation manifests itself in the mutation of a cysteine residue into serine, which disrupts the binding of EGFR protein to the third-generation targeted drugs, thereby failing to prevent the unilateral phosphorylation of EGFR and the activation of a downstream signaling pathway. Currently, there is yet no mature treatment method for two triple mutations that are prone to occur after resistance to osimertinib: de119/T790M/C797S and L858R/T790M/C797S.

Therefore, it is of great research significance to address the C797S mutation, overcome resistance to osimertinib, and provide safer and more effective EGFR inhibitors for patients.

### Summary of the Invention

The technical problem to be solved by the present invention is to provide a class of pyrimidine compounds, intermediates thereof, preparation methods therefor and use thereof, so as to overcome the shortcomings that existing EGFR inhibitors have a single structure and a poor inhibitory effect on C797S mutation. The compound of the present invention has a novel structure and a good inhibitory effect on EGFR Del19/T790M/C797S mutation, and is expected to treat and/or prevent a variety of diseases mediated by EGFR.

The present invention provides a compound as represented by formula I or a pharmaceutically acceptable salt thereof, 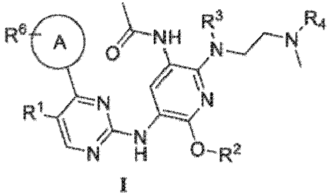
wherein ring A is 5- to 10-membered heteroaryl;
R¹ is H, halogen, C₁-C₄ alkyl, or C₁-C₄ alkyl substituted with halogen;
R² is C₁-C₄ alkyl or C₁-C₄ alkyl substituted with halogen;
R³ is H, C₁-C₄ alkyl, or C₁-C₄ alkyl substituted with halogen;
R⁴ is H, C₁-C₄ alkyl, or C₁-C₄ alkyl substituted with halogen; and
R⁶ is H, halogen, -CN, C₁-C₄ alkyl, or C₁-C₄ alkyl substituted with halogen.

In a certain scheme of the present invention, the halogen is independently F, Cl, Br, or I.

In a certain scheme of the present invention, the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl.

In a certain scheme of the present invention, the C₁-C₄ alkyl substituted with halogen is independently trifluoromethyl, -CH₂CH₂Cl, or -CH₂CF₃.

In a certain scheme of the present invention, the ring A is indolyl, indazolyl, benzothiazolyl, pyrimidinyl, or pyrazolyl.

In a certain scheme of the present invention, the pharmaceutically acceptable salt of the compound as represented by formula I can be a conventional salt in the art, for example, a mesylate salt of the compound as represented by formula I.

In a certain scheme of the present invention, the compound as represented by formula I can be

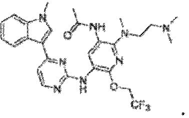

In a certain scheme of the present invention, the pharmaceutically acceptable salt of the compound as represented by formula I can be

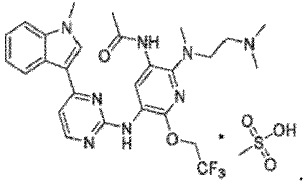

The present invention provides a compound as represented by formula II,

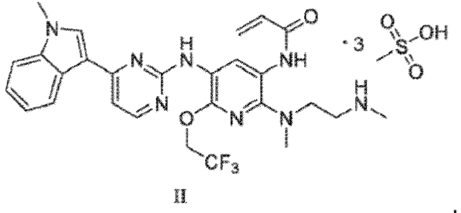

The present invention provides a compound as represented by formula III or a pharmaceutically acceptable salt thereof, 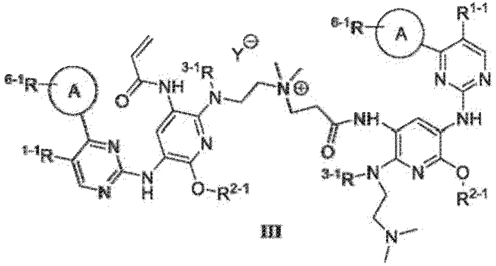
wherein ring A is 5- to 10-membered heteroaryl;
R¹⁻¹ is H, halogen, C₁-C₄ alkyl, or C₁-C₄ alkyl substituted with halogen;
R²⁻¹ is C₁-C₄ alkyl or C₁-C₄ alkyl substituted with halogen;
R³⁻¹ is H, C₁-C₄ alkyl, or C₁-C₄ alkyl substituted with halogen;
R⁶⁻¹ is H, halogen, -CN, C₁-C₄ alkyl, or C₁-C₄ alkyl substituted with halogen; and
Y^{Θ} is trifluoroacetate radical or mesylate radical.

In a certain scheme of the present invention, the halogen is independently F, Cl, Br, or I.

In a certain scheme of the present invention, the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl.

In a certain scheme of the present invention, the C₁-C₄ alkyl substituted with halogen is independently trifluoromethyl, -CH₂CH₂Cl, or -CH₂CF₃.

In a certain scheme of the present invention, the ring A is indolyl, indazolyl, benzothiazolyl, pyrimidinyl, or pyrazolyl.

In a certain scheme of the present invention, the pharmaceutically acceptable salt of the compound as represented by formula III can be a conventional salt in the art, for example, a trifluoroacetate salt of the compound as represented by formula III.

In a certain scheme of the present invention, the compound as represented by formula III can be

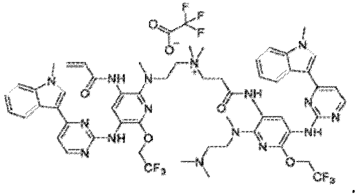

In a certain scheme of the present invention, the pharmaceutically acceptable salt of the compound as represented by formula III can be

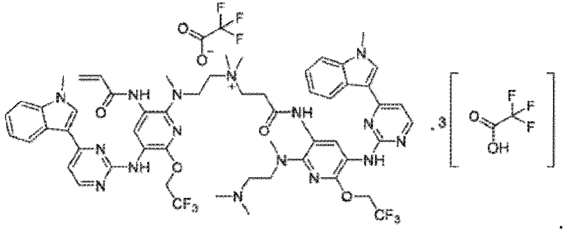

The present invention provides a compound as represented by formula VI or a pharmaceutically acceptable salt thereof, 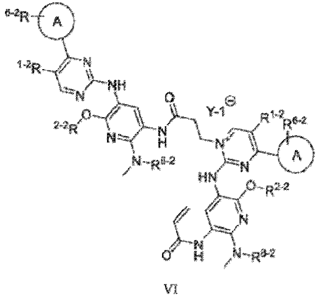
wherein ring A is 5- to 10-membered heteroaryl;
R¹⁻² is H, halogen, C₁-C₄ alkyl, or C₁-C₄ alkyl substituted with halogen;
R²⁻² is C₁-C₄ alkyl or C₁-C₄ alkyl substituted with halogen;
R⁶⁻² is H, halogen, -CN, C₁-C₄ alkyl, or C₁-C₄ alkyl substituted with halogen;
R⁸⁻² is C₁-C₄ alkyl or C₁-C₄ alkyl substituted with 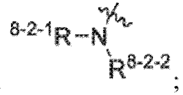
R⁸⁻²⁻¹ and R⁸⁻²⁻² are independently hydrogen, C₁-C₄ alkyl, or C₁-C₄ alkyl substituted with halogen; and 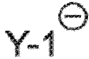
is trifluoroacetate radical or mesylate radical.

In a certain scheme of the present invention, the halogen is independently F, Cl, Br, or I.

In a certain scheme of the present invention, the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl.

In a certain scheme of the present invention, the C₁-C₄ alkyl substituted with halogen is independently trifluoromethyl, -CH₂CH₂Cl, or -CH₂CF₃.

In a certain scheme of the present invention, the ring A is indolyl, indazolyl, benzothiazolyl, pyrimidinyl, or pyrazolyl.

In a certain scheme of the present invention, the pharmaceutically acceptable salt of the compound as represented by formula VI can be a conventional salt in the art, for example, a trifluoroacetate salt of the compound as represented by formula VI.

In a certain scheme of the present invention, the compound as represented by formula VI can be

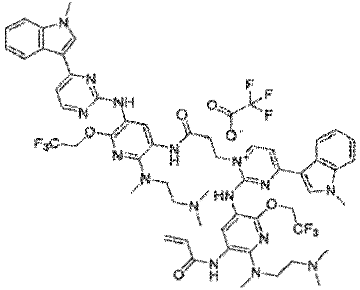

In a certain scheme of the present invention, the pharmaceutically acceptable salt of the compound as represented by formula VI can be

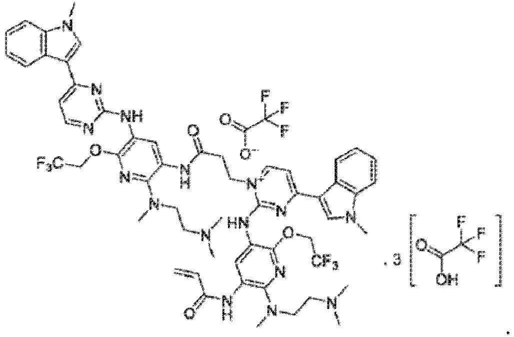

The present invention provides a compound as represented by formula V or a pharmaceutically acceptable salt thereof, 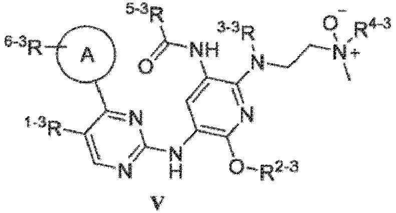
wherein ring A is 5- to 10-membered heteroaryl;
R¹⁻³ is H, halogen, C₁-C₄ alkyl or C₁-C₄ alkyl substituted with halogen;
R²⁻³ is C₁-C₄ alkyl or C₁-C₄ alkyl substituted with halogen;
R³⁻³ is H, C₁-C₄ alkyl, or C₁-C₄ alkyl substituted with halogen;
R⁴⁻³ is H, C₁-C₄ alkyl, or C₁-C₄ alkyl substituted with halogen;
R⁵⁻³ is 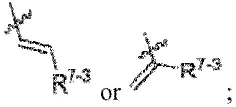
R⁶⁻³ is H, halogen, -CN, C₁-C₄ alkyl, or C₁-C₄ alkyl substituted with halogen; and
R⁷⁻³ is hydrogen, C₁-C₄ alkyl, or C₁-C₄ alkyl substituted with halogen.

In a certain scheme of the present invention, the halogen is independently F, Cl, Br, or I.

In a certain scheme of the present invention, the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl.

In a certain scheme of the present invention, the C₁-C₄ alkyl substituted with halogen is independently trifluoromethyl, -CH₂CH₂Cl, or -CH₂CF₃.

In a certain scheme of the present invention, the ring A is indolyl, indazolyl, benzothiazolyl, pyrimidinyl, or pyrazolyl.

In a certain scheme of the present invention, the pharmaceutically acceptable salt of the compound as represented by formula V can be a conventional salt in the art, for example, a mesylate salt of the compound as represented by formula V.

In a certain scheme of the present invention, the compound as represented by formula V can be

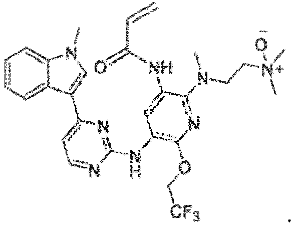

In a certain scheme of the present invention, a pharmaceutically acceptable salt of the compound as represented by formula V can be

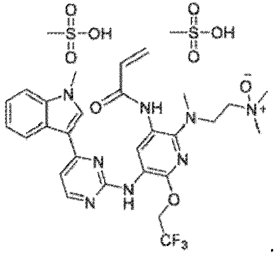

The present invention further provides a preparation method for a compound as represented by formula I-a, comprising the step of salt-forming reaction of the compound as represented by formula I with methanesulfonic acid in a ketone solvent to afford the compound as represented by formula I-a; 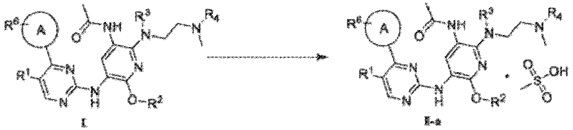 wherein each group is as defined above.

In a certain scheme of the present invention, the ketone solvent can be a conventional solvent in the art, for example, acetone.

In a certain scheme of the present invention, the salt-forming reaction can be at a temperature which can be a conventional temperature in the art, for example, 40-60°C, for example, 50°C.

In a certain scheme of the present invention, the salt-forming reaction is proceeded till reaction termination, and preferably, the salt-forming reaction can be for 45-90 minutes, preferably 60 minutes.

The preparation method for the compound as represented by formula I-a can further comprise the step of: amide reaction of a compound M with acetyl chloride in an organic solvent in the presence of a base to afford the compound as represented by formula I;

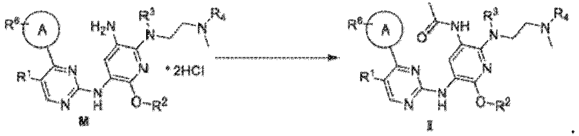

In a certain scheme of the present invention, the organic solvent can be a conventional organic solvent for such reactions in the art, for example, dichloromethane.

In a certain scheme of the present invention, the base can be a conventional base for such reactions in the art, for example, triethylamine.

In a certain scheme of the present invention, the amide reaction can be at a temperature which can be a conventional temperature for such reactions in the art, for example, -10°C-0°C.

In a certain scheme of the present invention, the amide reaction can be subject to a post-treatment which can be a conventional post-treatment for such reactions in the art; for example, the amide reaction can be subject to the post-treatment by extracting and washing with dichloromethane and saturated sodium bicarbonate, followed by back extracting and washing with dichloromethane.

The present invention further provides a preparation method for a compound as represented by formula I, comprising the step of: amide reaction of a compound M with acetyl chloride in an organic solvent under the action of a base to afford the compound as represented by formula I; 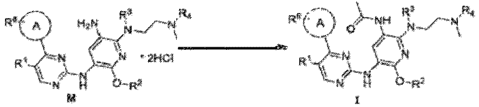 wherein each group is as defined above; and the amide reaction can be under the reaction conditions as defined above.

The present invention further provides a preparation method for a compound as represented by formula II, comprising the step of reaction of a compound P with methanesulfonic acid in a ketone solvent to afford the compound as represented by formula II;

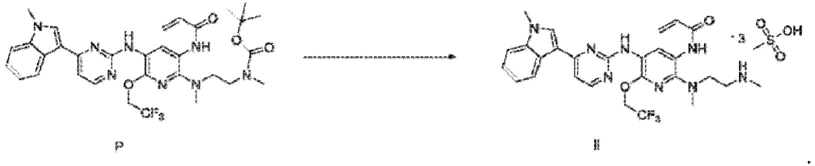

In a certain scheme of the present invention, the ketone solvent can be a conventional solvent in the art, for example, acetone.

In a certain scheme of the present invention, the reaction can be at a temperature which can be a conventional temperature for such reactions in the art, for example, 30-50°C, for example, 35°C.

In a certain scheme of the present invention, the reaction is proceeded till reaction termination and preferably, the reaction can be for 2-6 hours, preferably 4 hours.

In a certain scheme of the present invention, the reaction can be subject to a post-treatment which can be a conventional post-treatment for such reactions in the art; for example, beating with ethyl acetate and drying at 50°C.

The preparation method for the compound as represented by formula II can further comprise:
(1) amination reaction of a compound P-4 with N,N'-dimethylethylenediamine in N,N-dimethylformamide to afford a compound P-3;
(2) reaction of the compound P-3 with di-tert-butyl dicarbonate in dichloromethane in the presence of triethylamine to afford a compound P-2;
(3) reduction of the compound P-2 with sodium hydrosulfite in ethanol and water to afford a compound P-1; and
(4) reaction of the compound P-1 with 3-chloropropionyl chloride in dichloromethane in the presence of triethylamine to afford the compound P;

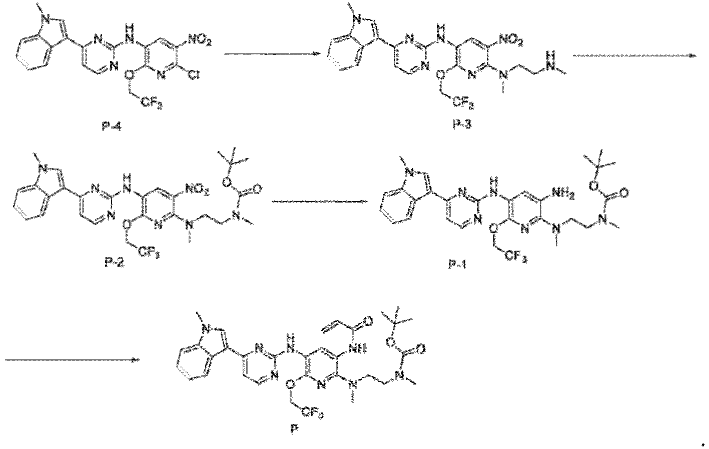

The present invention further provides a preparation method for a compound as represented by formula III or a pharmaceutically acceptable salt thereof, comprising the steps of reaction of an acid with a compound N in a solvent, and optionally purification by high performance liquid chromatography to afford the compound as represented by formula III or a pharmaceutically acceptable salt thereof; 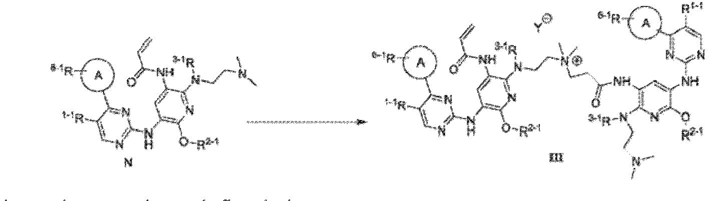 wherein each group is as defined above.

In a certain scheme of the present invention, the solvent is selected from one or a mixture of acetone and water.

In a certain scheme of the present invention, the acid can be methanesulfonic acid and trifluoroacetic acid.

In a certain scheme of the present invention, the reaction can be at a temperature which can be a conventional temperature for such reactions in the art, for example, 50-55°C.

In a certain scheme of the present invention, the reaction is proceeded till reaction termination, and preferably, the reaction can be for 36-72 hours, preferably 48 hours.

In a certain scheme of the present invention, the reaction can be subject to a post-treatment which can be a conventional post-treatment for such reactions in the art; for example, the reaction can be subject to the post-treatment by beating with tetrahydrofuran and acetonitrile.

In a certain scheme of the present invention, the purification by high performance liquid chromatography can be under the following conditions: mobile phase A: a solution of 0.1% trifluoroacetic acid in water; mobile phase B: acetonitrile; flow rate: 120mL/min; and wavelength: 332nm.

The present invention further provides a preparation method for a compound as represented by formula VI or a pharmaceutically acceptable salt thereof, comprising the steps of addition reaction of a compound N-1 by heating to 70-90°C in a solvent, and optionally purification by high performance liquid chromatography to afford the compound as represented by formula VI or a pharmaceutically acceptable salt thereof; 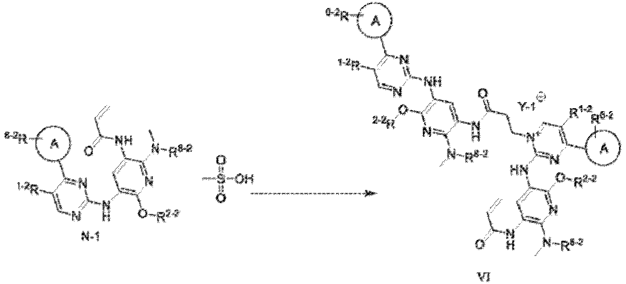 wherein each group is as defined above.

In a certain scheme of the present invention, the solvent can be a conventional solvent for such reactions in the art, for example, water.

In a certain scheme of the present invention, the addition reaction can be at a temperature which can be a conventional temperature for such reactions in the art, for example, 70-90°C, preferably 80°C.

In a certain scheme of the present invention, the purification by high performance liquid chromatography can be under the following conditions: mobile phase A: a solution of 0.1% trifluoroacetic acid in water; mobile phase B: a solution of 0.1% trifluoroacetic acid in acetonitrile; flow rate: 1.5 mL/min.

The present invention further provides a preparation method for a compound as represented by formula V or a pharmaceutically acceptable salt thereof, comprising the steps of: oxidation reaction of a compound N-2 in an organic solvent in the presence of an oxidizing agent H₂O₂, and further optionally salt-forming reaction to afford the compound as represented by formula V or a pharmaceutically acceptable salt thereof; 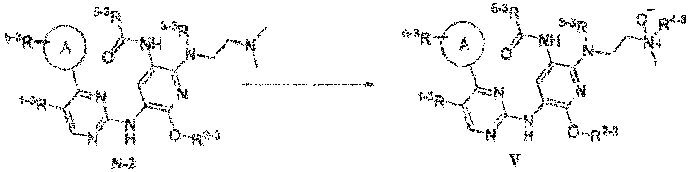 wherein each group is as defined above.

In a certain scheme of the present invention, the organic solvent can be a conventional organic solvent for such reactions in the art, for example, ethanol.

In a certain scheme of the present invention, the oxidation reaction can be at a temperature of 40-60°C, preferably 50°C.

In a certain scheme of the present invention, the salt-forming reaction can be under the following conditions: directly salt-forming by an oxidation product of the compound N-2 with methanesulfonic acid in a mixed solvent of acetone and water at a temperature of 20-30°C.

The present invention further provides a pharmaceutical composition, comprising a therapeutically effective amount of a substance X and pharmaceutically acceptable supplementary materials;
the substance X is any one of the following substances:
(1) the compound as represented by formula I as described above or a pharmaceutically acceptable salt thereof;
(2) the compound as represented by formula II as described above;
(3) the compound as represented by formula III as described above or a pharmaceutically acceptable salt thereof;
(4) the compound as represented by formula VI as described above or a pharmaceutically acceptable salt thereof;
or (5) the compound as represented by formula V as described above or a pharmaceutically acceptable salt thereof.

The present invention further provides use of a substance X or the pharmaceutical composition as described above in preparation of an EGFR inhibitor, wherein the EGFR inhibitor is an inhibitor of EGFR Del19/T790M/C797S mutation; wherein in the use, the EGFR inhibitor can be used in mammals *in vivo*; and it can also be used *in vitro* mainly for experimental purposes, for example: it is used as a standard sample or a control sample to provide comparison, or it is prepared into a kit according to a conventional method in the art to provide rapid detection for EGFR-inhibiting effects;
wherein the substance X is any one of the following substances:
(1) the compound as represented by formula I as described above or a pharmaceutically acceptable salt thereof;
(2) the compound as represented by formula II as described above;
(3) the compound as represented by formula III as described above or a pharmaceutically acceptable salt thereof;
(4) the compound as represented by formula VI as described above or a pharmaceutically acceptable salt thereof;
or (5) the compound as represented by formula V as described above or a pharmaceutically acceptable salt thereof.

The present invention further provides use of a substance X or the pharmaceutical composition as described above in preparation of a medicament for treatment and/or prevention of a disease mediated by EGFR or for treatment and/or prevention of a cancer;
The disease mediated by EGFR is a disease mediated by EGFR Del19/T790M/C797S mutation;
the substance X is any one of the following substances:
(1) the compound as represented by formula I as described above or a pharmaceutically acceptable salt thereof;
(2) the compound as represented by formula II as described above;
(3) the compound as represented by formula III as described above or a pharmaceutically acceptable salt thereof;
(4) the compound as represented by formula VI as described above or a pharmaceutically acceptable salt thereof;
or (5) the compound as represented by formula V as described above or a pharmaceutically acceptable salt thereof.

The disease mediated by EGFR as described above can be a disease resistant to the first-, second- and third-generation EGFR inhibitors, wherein the first-, second- and third-generation EGFR inhibitors can be selected from gefitinib, erlotinib, icotinib, afatinib, dacomitinib, and osimertinib.

The disease mediated by EGFR as described above can be a disease mediated by EGFR Del19/T790M/C797S mutation.

The disease mediated by EGFR as described above can be a cancer.

The cancer as described above can be selected from one or more of colon cancer, pancreatic cancer, breast cancer, prostate cancer, lung cancer, brain cancer, ovarian cancer, cervical cancer, testicular cancer, kidney cancer, head or neck cancer, bone cancer, skin cancer, rectal cancer, liver cancer, colorectal cancer, non-small cell lung cancer, small cell lung cancer, lung adenocarcinoma, lung squamous carcinoma, esophageal cancer, gastric cancer, pancreatic cancer, thyroid cancer, bladder cancer, lymphoma, glioma, glioblastoma, gastrointestinal stromal tumor, bile duct cancer, endometrial cancer, multiple myeloma, leukemia, and melanoma.

In addition to the aforementioned, when used in the description and claims of the present application, unless otherwise specified, the following terms have the meanings shown below.

The term "pharmaceutically acceptable salt" refers to a salt obtained by reaction of a compound with a pharmaceutically acceptable (relatively nontoxic, safe, and suitable for patients) acid or base. When a compound contains a relatively acidic functional group, a base addition salt can be obtained by contacting a free form of the compound with a sufficient amount of a pharmaceutically acceptable base in a suitable inert solvent. The pharmaceutically acceptable base addition salts include, but are not limited to, sodium salts, potassium salts, calcium salts, aluminum salts, magnesium salts, bismuth salts, ammonium salts, etc. When a compound contains a relatively basic functional group, an acid addition salt can be obtained by contacting a free form of the compound with a sufficient amount of a pharmaceutically acceptable acid in a suitable inert solvent. The pharmaceutically acceptable acids include inorganic acids and organic acids. For details, see Berge et al., "Pharmaceutical Salts," Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

In the present application, "pharmaceutical composition" refers to a preparation comprising the compound of the present invention and a medium generally accepted in the art for delivering a biologically active compound to a mammal (for example, a human). The medium comprises a pharmaceutically acceptable carrier. The purpose of the pharmaceutical composition is to facilitate the administration to an organism and facilitate the absorption of an active ingredient to exert biological activity.

In the present application, "pharmaceutically acceptable" refers to a substance (such as a pharmaceutical supplementary material) that does not affect the biological activity or properties of the compound of the present invention and is relatively non-toxic, that is, the substance can be administered to a subject without causing an adverse effect or interacting with any component included in a composition in an adverse manner.

The term "pharmaceutical supplementary materials" or "pharmaceutically acceptable carriers" refers to excipients and additives used during the production of drugs and preparation of prescriptions, and refers to all substances other than active ingredients included in a pharmaceutical preparation. See Pharmacopoeia of the People's Republic of China (2015 Edition), Part IV or Handbook of Pharmaceutical Excipients (Raymond C Rowe, 2009 Sixth Edition). A supplementary material is mainly used for providing a safe, stable and functional pharmaceutical composition, and can further provide a method to enable an active ingredient to dissolve at a desired rate after administration to a subject, or promote the effective absorption of the active ingredient after administration of the composition to the subject. The pharmaceutical supplementary material can be an inert filler or can provide a certain function, for example, for stabilizing the overall pH value of the composition or preventing the degradation of an active ingredient of the composition. The pharmaceutical supplementary material can include one or more of the following supplementary materials: binders, suspending agents, emulsifiers, diluents, fillers, granulating agents, adhesives, disintegrants, lubricants, anti-adhesive agents, glidants, wetting agents, gelling agents, absorption delaying agents, dissolution inhibitors, enhancers, adsorbents, buffers, chelating agents, preservatives, colorants, flavorings, and sweeteners.

A pharmaceutical composition of the present invention can be prepared according to the disclosure by using any method known to those skilled in the art. For example, conventional mixing, dissolving, granulating, emulsifying, grinding, encapsulating, embedding or freeze-drying processes.

When used as a medicament, the pyrimidine compounds as represented by formula I, formula II, formula III, formula VI, and formula V or pharmaceutically acceptable salts thereof can be administered in any form of a pharmaceutical composition. These compositions can be prepared according to a method well known in the pharmaceutical field and can be administered by a variety of approaches, depending on the desired local or systemic treatment and the area to be treated. Administration can be topical (including epidermal and transdermal, ocular, and mucosal, including intranasal, vaginal and rectal delivery), pulmonary (e.g., by inhalation or insufflation of powder or aerosol, including by a sprayer; intratracheal or intranasal), oral (solid and liquid preparations), or parenteral administration forms. Examples of solid oral preparations include, but are not limited to, powders, capsules, caplets, soft capsules, and tablets. Examples of liquid preparations for oral or mucosal administration include, but are not limited to, suspensions, emulsions, elixirs and solutions. Examples of topical preparations include, but are not limited to, emulsions, gels, ointments, creams, patches, pastes, foams, lotions, drops, or serum preparations. Examples of preparations for parenteral administration include, but are not limited to, solutions for injection, dry preparations that may be dissolved or suspended in a pharmaceutically acceptable carrier, suspensions for injection, and emulsions for injection. Pharmaceutical compositions and preparations for external administration can include transdermal patches, salves, emulsions, ointments, gels, drops, suppositories, sprays, liquids, and powders. Examples of other suitable preparations of the pharmaceutical composition include, but are not limited to, eye drops and other ophthalmic preparations; aerosols: such as nasal sprays or inhalants. Oral administration can include dosage forms formulated for administering once a day or twice a day (BID). Parenteral administration includes intravenous, intraarterial, subcutaneous, intraperitoneal, intramuscular administration or by injection or infusion; or intracranial administration such as intrathecal or intraventricular administration. Parenteral administration may be in the form of a single bolus dose or can be achieved by a continuous infusion pump. Conventional pharmaceutical carriers, water, powdered or oily substrates, thickening agents, etc. may be necessary or desired. The pharmaceutical composition comprising the present invention can further be in a controlled or delayed release dosage form (for example, liposomes or microspheres).

The term "treatment" refers to a therapeutic therapy or a palliative measure. When referring to a specific condition, the treatment refers to: (1) palliation of one or more biological manifestations of a disease or a condition; (2) interference with (a) one or more points in the biological cascade resulting in or causing the condition or (b) one or more biological manifestations of the condition; (3) amelioration of one or more symptoms, effects or side effects related to the condition, or one or more symptoms, effects or side effects related to the condition or the treating thereof; or (4) slowing of the development of the condition or one or more biological manifestations of the condition. "Treatment" can also refer to prolonging the survival in comparison to an expected survival without treatment.

The term "prevention" refers to reducing the risk of acquiring or developing a disease or a disorder.

The term "therapeutically effective amount" refers to an amount of a compound sufficient to effectively treat the disease or condition described herein when administered to a patient. The "therapeutically effective amount" will vary according to compounds, conditions and their severities, as well as the ages of the patients to be treated, but may be adjusted as necessary by those skilled in the art.

The term "patient" refers to any animal, preferably mammals, and most preferably humans, to which the compound or composition is to be or has been administered according to the embodiments of the present invention. The term "mammal" includes any mammal. Examples of mammals include, but are not limited to, cows, horses, sheep, pigs, cats, dogs, mice, rats, rabbits, guinea pigs, monkeys, humans, etc., most preferably humans.

Unless otherwise stated, the following definitions used herein shall apply. For the purposes of the present invention, chemical elements are consistent with the CAS edition of the Periodic Table of the Elements and the Handbook of Chemistry and Physics, 75th edition, 1994. In addition, general principles of organic chemistry can be referred to the description in "Organic Chemistry," Thomas Sorrell, University Science Books, Sausalito: 1999 and "March's Advanced Organic Chemistry" by Michael B. Smith and Jerry March, John Wiley & Sons, New York: 2007, the contents of which are incorporated herein by reference in their entireties.

In this description, groups and their substituents can be selected by those skilled in the art to provide stable structure moieties and compounds. When a substituent is described by a conventional chemical formula written from left to right, the substituent also includes a chemically equivalent substituent obtained by writing a structural formula from right to left.

Certain chemical groups defined herein are preceded by simplified symbols to represent the total number of carbon atoms present in the groups. For example, C₁-C₄ alkyl or C₁₋₄ alkyl refers to alkyl having a total of 1, 2, 3 or 4 carbon atoms as defined below. The total number of carbon atoms in the simplified symbol excludes carbons that may be present in a substituent of said group.

Herein, the numerical range defined in the substituent, such as 0-4, 1-4, 1-3, etc., indicates the integers within the range, such as 1-6 including 1, 2, 3, 4, 5, 6.

The term "comprise" is an open-ended expression, namely including the contents specified in the present invention, without excluding the contents of other aspects.

The term "substituted" means that any one or more hydrogen atoms on a specified atom are substituted with a substituent, as long as the valence state of the specified atom is normal and the substituted compound is stable.

Generally speaking, the term "substituted" means that one or more hydrogen atoms in a given structure are substituted with a specified substituent. Further, when the group is substituted with more than 1 substituent mentioned above, the substituents are independent of each other, that is, the more than 1 substituent can be different from each other or can be the same. Unless otherwise indicated, a substituent group can substitute at each substitutable position of the substituted group. When more than one position in a given structural formula can be substituted with one or more substituents selected from specific groups, the substituents can substitute identically or differently at each position.

In various parts of this description, substituents of the compounds disclosed by the present invention are disclosed according to group types or ranges. It should be particularly noted that the present invention includes each independent subcombination of various members of these group types and ranges. The term "Cₓ-C_{y} alkyl" or "C_{x-y} alkyl" refers to a linear or branched saturated hydrocarbon containing x to y carbon atom(s). For example, the term "C₁-C₆ alkyl" or "C₁₋₆ alkyl" specifically refers to independently disclosed methyl, ethyl, C₃ alkyl, C₄ alkyl, C₅ alkyl, and C₆ alkyl; "C₁-C₄ alkyl" specifically refers to independently disclosed methyl, ethyl, C₃ alkyl (i.e., propyl, including n-propyl and isopropyl), and C₄ alkyl (i.e., butyl, including n-butyl, isobutyl, sec-butyl and tert-butyl).

The terms "moiety," "structural moiety," "chemical moiety," "group" and "chemical group" used herein refer to specific fragments or functional groups in a molecule. A chemical moiety is generally considered as a chemical entity embedded in or attached to a molecule.

When an enumerated substituent does not indicate by which atom it is attached to a general chemical structure (including but not specifically mentioned compounds), such substituent can be bonded by any atom thereof. A combination of substituents and/or variants thereof is permitted only when such combination results in a stable compound.

When an enumerated group does not expressly indicate that it has a substituent, such group is simply unsubstituted. For example, when "C₁-C₄ alkyl" is not preceded by "substituted or unsubstituted," it only refers to "C₁-C₄ alkyl" itself or "unsubstituted C₁-C₄ alkyl."

In various parts of the present invention, linking substituents are described. When a linking group is clearly required for the structure, the Markush variable enumerated for that group should be understood as a linking group. For example, if a linking group is required for the structure and the Markush group definition for the variable enumerates "alkyl," it will be understood that the "alkyl" represents an attached alkylene group.

In some specific structures, when an alkyl group is expressly represented as a linking group, then the alkyl group represents an attached alkylene group, for example, C₁₋₄ alkyl in the group "halo-C₁₋₄ alkyl" should be understood as C₁₋₄ alkylene.

The term "halogen" refers to fluoro, chloro, bromo, or iodo, especially F or Cl.

In the present application, the term "alkyl," as a group or a moiety of another group (for example, as used in groups such as haloalkyl, deuteroalkyl, etc.), refers to branched and linear saturated aliphatic hydrocarbon groups having a specified number of carbon atoms, which consists only of carbon atoms and hydrogen atoms and has, for example, 1 to 12 (preferably 1 to 8, more preferably 1 to 6, and more preferably 1 to 4) carbon atoms, and is attached to the remainder of the molecule by a single bond, wherein propyl is C₃ alkyl (including isomers, for example, n-propyl or isopropyl); butyl is C₄ alkyl (including isomers, for example, n-butyl, sec-butyl, isobutyl, or tert-butyl); pentyl is C₅ alkyl (including isomers, for example, n-pentyl, 1-methyl-butyl, 1-ethylpropyl, 2-methyl-1-butyl, 3-methyl-1-butyl, isopentyl, tert-pentyl, or neopentyl); hexyl is C₆ alkyl (including isomers, for example, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl). For example, the alkyl includes, but is not limited to, methyl, ethyl, n-propyl, isopropyl, n-butyl, isobutyl, sec-butyl, tert-butyl, n-pentyl, 2-methylbutyl, 2,2-dimethylpropyl, n-hexyl, n-heptyl, 2-methylhexyl, 3-methylhexyl, n-octyl, nonyl, and decyl, and other similar alkyl.

The term "heteroaryl" refers to an aromatic group containing heteroatoms, preferably an aromatic 5- to 6-membered monocyclic ring or 9- to 10-membered bicyclic ring containing 1, 2 or 3 heteroatoms independently selected from nitrogen, oxygen and sulfur, for example, furyl, pyridyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzisothiazolyl, benzoxazolyl, benzisoxazolyl, quinolinyl, isoquinolinyl, etc.

Unless otherwise stated, the present invention adopts traditional methods of mass spectrometry and elemental analysis, and each step and condition can be referred to the conventional operating steps and conditions in the art.

Unless otherwise indicated, the present invention adopts the standard nomenclature and standard laboratory procedures and techniques for analytical chemistry, synthetic organic chemistry, and photology. In some cases, standard techniques are used for chemical synthesis, chemical analysis, and light-emitting device performance testing.

In addition, it should be noted that, unless otherwise clearly stated, the description method "...independently" used in the present invention should be understood in a broad sense, meaning that various described individuals are independent of each other and can be independently the same or different specific groups. In more detail, the description method "...independently" can represent that in different groups, the specific options expressed by the same symbols do not affect each other; and it can also represent that in the same group, the specific options expressed by the same symbols do not affect each other.

Unless otherwise specified, all technical and scientific terms used herein have their standard meanings in the art to which the claimed subject matters pertain. If there are multiple definitions for a term, the definition herein shall prevail.

On the basis of not violating common knowledge in the art, the above-mentioned preferred conditions may be combined arbitrarily to namely obtain various preferred examples of the present invention.

The reagents and raw materials used in the present invention are all commercially available.

The positive and progressive effects of the present invention lie in that: the pyrimidine compound has a good inhibitory effect on EGFR Del19/T790M/C797S mutation, and is expected to treat and/or prevent a variety of diseases mediated by EGFR, for example, the diseases resistant to the first-, second- and third- generation EGFR inhibitors, for another example, the diseases mediated by EGFR Del19/T790M/C797S mutation.

### Detailed Description of Embodiments

The present invention is further illustrated below by means of embodiments, but the present invention is not hereby limited to the scope of the embodiments. For the experimental methods without specific conditions specified in the following embodiments, selection shall be made according to conventional methods and conditions or according to product instructions.

### Embodiment 1

### Synthesis processes of compound 1-1 and compound 1-2

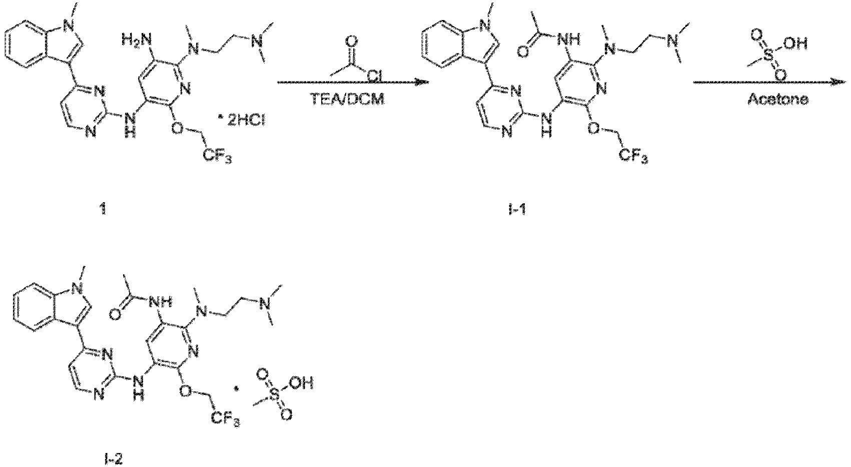

### Preparation of the compound I-1:

To a reaction flask were added a compound 1 (50.0g, 1.0eq) and dichloromethane (425mL), followed by stirring and addition of triethylamine (17.2g, 1.5eq); an internal temperature of the reaction solution was controlled within the range of -10°C-0°C, and a solution of acetyl chloride (10.0g, 1.5eq) in dichloromethane (325mL) was added dropwise. After the dropwise addition was completed, and the reaction was monitored to be completed, suction filtration was performed to afford a solid wet product which was transferred to the reaction flask, and extracted and washed using the added dichloromethane (500mL) and saturated sodium bicarbonate (500mL); the pH of the aqueous phase was measured to be 7-8, and liquid separation was performed; the aqueous phase was back extracted once again with dichloromethane (500mL); organic phases were combined and washed once with purified water (500mL), and the liquid separation was performed; and the organic phase was concentrated under reduced pressure to afford 55g of a solid. To the solid was added tetrahydrofuran (40mL), and after the solid was stirred to be completely dissolved at room temperature, n-heptane (120mL) was added dropwise therein; after the dropwise addition was completed, stirring was continued for 1 hour; suction filtration was performed, and the filter cake was dried to afford 36.5g of an off-white solid with a purity of 99.7% and a yield of 77%.

MS m/z: 557.6[M+H]⁺.

¹H NMR (400 MHz, DMSO-*d₆*) δ 9.78 (s, 1H), 8.56 (s, 1H), 8.41 (s, 1H), 8.27 (t, J = 4.9 Hz, 2H), 8.12 (s, 1H), 7.52 (d, J = 8.2 Hz, 1H), 7.30 - 7.10 (m, 3H), 4.98 (q, J = 9.1 Hz, 2H), 3.89 (s, 3H), 3.19 (t, J = 6.6 Hz, 2H), 2.87 (s, 3H), 2.48 (d, J = 6.6 Hz, 4H), 2.24 (s, 6H), 2.07 (s, 3H).

### Preparation of compound 1-2:

To a reaction flask were added I-1 (3.0g, 1.0eq) and acetone (21mL), followed by stirring and heating to 50°C; a mixed solution of methanesulfonic acid (518mg, 0.99eq) in acetone (9mL) was added dropwise; after the dropwise addition was completed, stirring was continued for 1 hour under heat preservation; and the mixture was cooled to 20°C, subjected to suction filtration, and dried under reduced pressure at 50°C to afford 2.9g of an off-white solid with a purity of 99.9% and a yield of 82.5%.

MS m/z: 557.6[M+H]⁺.

¹H NMR (400 MHz, DMSO-*d₆*) δ 9.55 (s, 1H), 9.21 (s, 1H), 8.46 (s, 1H), 8.41 (s, 1H), 8.30 (d, J = 5.4 Hz, 2H), 8.17 (s, 1H), 7.53 (d, J = 8.2 Hz, 1H), 7.30 - 7.14 (m, 3H), 5.01 (q, J = 9.1 Hz, 2H), 3.89 (s, 3H), 3.64 (t, J = 6.2 Hz, 2H), 2.85 (d, J = 11.3 Hz, 9H), 2.44 (d, J = 1.0 Hz, 3H), 2.17 (s, 3H), 2.09 (s, 3H).

¹³C-NMR ((400 MHz, DMSO-*d₆*) δ169.97, 162.03, 160.14, 157.20, 148.47, 147.79, 137.66, 133.25, 131.46, 128.25, 125.49, 125.46, 122.73, 122.12, 121.82, 120.82, 119.96, 118.24, 117.00, 112.32, 110.43, 107.24, 62.32, 54.11, 46.23, 42.63, 40.16, 40.08, 32.97, 23.27.

### Embodiment 2

### Synthesis process of compound II

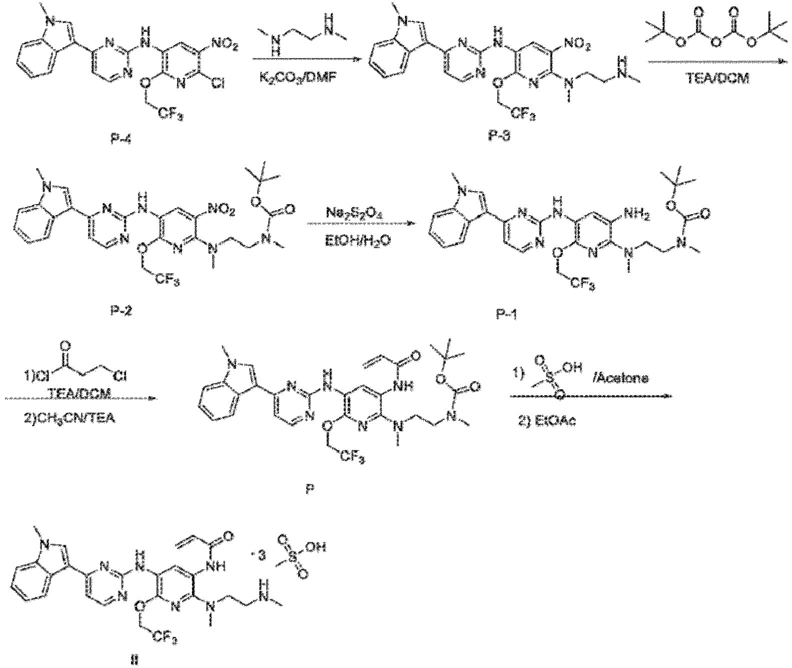

### Preparation of P-3

To a reaction flask were successively added potassium carbonate (95.7g, 1.5eq), N,N'-dimethylethylenediamine (204.0g, 5.0eq) and N,N-dimethylformamide (3L), followed by stirring and internal heating to 50°C; the temperature was controlled within the range of 50-70°C, and P-4 (300.0g, 1.0eq) was added in batches; after the addition was completed, stirring was continued; and after raw materials were monitored to react completely in a liquid phase manner, to the reaction system was added water (3L). After the addition was completed, the temperature was reduced to 20-25°C, suction filtration was performed, and the filter cake was dried under forced air for 20 hours at 50°C to afford 183g of a yellow solid powder with a yield of 74.7% and a liquid phase purity of 99.3%.

MS m/z: 531.2[M+H]⁺.

¹H NMR (400 MHz, DMSO-*d₆*) δ 8.66 (s, 1H), 8.47 (s, 1H), 8.36 - 8.20 (m, 3H), 7.50 (d, J = 8.2 Hz, 1H), 7.31 - 7.14 (m, 2H), 7.07 (t, J = 7.4 Hz, 1H), 5.11 (q, J = 8.9 Hz, 2H), 3.92 (t, J = 6.9 Hz, 2H), 3.86 (s, 3H), 3.18 (t, J = 7.0 Hz, 2H), 2.89 (s, 3H), 2.54 (s, 3H), 1.22 (s, 1H).

### Preparation of P-2

To a reaction flask were successively added P-3 (183g, 1.0eq) and dichloromethane (1.1L), followed by stirring and addition of triethylamine (52.4g, 1.5eq); the reaction system was cooled to 0°C, the internal temperature was controlled within the range of 0-10°C, and a mixed solution of di-tert-butyl dicarbonate (90.3g, 1.2eq) in dichloromethane (360mL) was added dropwise; after the dropwise addition was completed, the temperature was raised to 20-30°C, stirring was continued, and raw materials were monitored to react completely in a liquid phase manner; to the system was added water (1.8L), and an organic phase was separated out after stirring and back extracted once again with water (1.8L); liquid separation was performed, and the organic phase was taken and concentrated under reduced pressure to afford a crude product; then, ethanol (540mL) was added, followed by beating for 30 minutes at room temperature and suction filtration, and the solid was dried under forced air for 20 hours at 50°C to afford 210g of a yellow solid powder with a yield of 96.5% and a liquid phase purity of 100%.

MS m/z: 631.3[M+H]⁺.

¹H NMR (400MHz, DMSO-*d₆*) δ 8.65 (d, 1H), 8.45 (s, 1H), 8.07-8.41(m, 3H), 7.51(d, 1H), 7.14-7.30(m, 2H), 7.07(t, 1H), 4.93-5.25(m, 2H), 3.89(s, 3H), 3.78-3.90(m, 2H), 3.48-3.55(m, 2H), 2.89(s, 3H), 2.84(d, 3H), 1.35(d, 9H).

### Preparation of P-1

To a reaction flask were added P-2 (210g, 1.0eq), ethanol (1.05L) and water (1.05L), followed by stirring and internal heating to 45°C, and sodium hydrosulfite (1452g, 18eq) was added in batches; after the addition was completed, raw materials were monitored to react completely; the system was cooled to room temperature, followed by standing for layering to separate out an organic phase; to the aqueous phase was added water (1L) for dissolution and clarification, and then the mixture was back extracted twice with ethyl acetate (0.5L×2); organic phases were combined, evaporated in a rotary manner under reduced pressure, and spin-dried to afford a crude product; to the crude product was added dichloromethane (1.5L), suction filtration was performed over a diatomite medium after dissolution and clarification, and the filtrate was concentrated under reduced pressure to afford 200g of a brown solid with a yield of 100% and a liquid phase purity of 87.3 %.

MS m/z: 601.3[M+H]⁺.

¹H NMR(400MHz, DMSO-*d₆*) δ 8.22-8.38 (m, 3H), 7.98(s, 1H), 7.64(s, 1H), 7.52(d, 1H), 7.21-7.30(m,1H), 7.04-7.18(m, 2H), 4.80-4.95(m, 2H), 4.48(s, 2H), 3.87(s, 3H), 3.20-3.40(m, 2H), 2.78-2.82(m, 2H), 2.73(s, 3H), 1.39(s, 9H).

### Preparation of P

To a reaction flask were successively added P-1 (200g), dichloromethane (2L) and triethylamine (67g, 2.0eq), followed by stirring and internal cooling to 5°C, and a mixed solution of 3-chloropropionyl chloride (63g, 1.5eq) in dichloromethane (400mL) was added dropwise; after the dropwise addition was completed, the temperature was raised to 20-30°C for reaction for 1 hour; and raw materials were monitored to react completely, and a reaction liquid was concentrated to dryness under reduced pressure.

To the concentrate were added acetonitrile (1.2L) and triethylamine (167g, 5.0eq), followed by stirring and heating to 80°C for reaction for 2 hours; the mixture was monitored till there was no intermediate product, and cooled to 20-30°C; to the system was added water (2L), and the mixture was extracted twice with dichloromethane (1.2L×2), and organic phases were combined, concentrated under reduced pressure and spin-dried to afford a brown solid; ethanol (1.0L) was added, followed by heating to 75°C, beating and natural cooling to 20-30°C, and suction filtration was performed to afford a yellow solid crude product which was dried under forced air at 50°C to be 100g, with a purity of 97.9% as measured by liquid phase detection.

To the above sample was added acetonitrile (1.5L) again, followed by heating to 75°C for dissolution and clarification, stirring for half an hour and dropwise addition of water (1.5L); after the addition was completed, the mixture was cooled to 20-30°C, stirred for half an hour, subjected to suction filtration, and dried under forced air at 50°C to afford 90g of a yellow solid powder with a yield of 41.3% and a liquid phase purity of 99.5%.

MS m/z: 655.3[M+H]⁺.

¹H NMR (400 MHz, DMSO-d6) δ 9.56 (s, 1H), 8.41 - 8.22 (m, 4H), 8.11 (s, 1H), 7.51 (d, J = 8.1 Hz, 1H), 7.28 - 7.11 (m, 3H), 6.60 (dd, J = 17.0, 10.2 Hz, 1H), 6.25 (dd, J = 17.1, 2.0 Hz, 1H), 5.79 - 5.71 (m, 1H), 5.01 (d, J = 16.1 Hz, 2H), 3.89 (s, 3H), 3.35-3.38(m, 5H), 2.93 (s, 3H), 2.80 - 2.76 (m, 2H), 1.38 (s, 9H).

### Preparation of compound II

To a reaction flask were added P (90g, 1.0eq) and acetone (1.1V), followed by stirring and heating to 35°C, and a mixed solution of methanesulfonic acid (105.7g, 8.0eq) in acetone (270mL) was added dropwise; after the dropwise addition was completed, reaction was stirred and continued under heat preservation for 4 hours; raw materials were monitored to react completely in a liquid phase manner, the mixture was cooled to room temperature and subjected to suction filtration to afford 140g of a crude wet product as a yellow solid with a liquid phase purity of 99.7%.

140g of the above crude product was added to the reaction flask, followed by addition of ethyl acetate (0.7L), heating to 65°C and beating under reflux for 1 hour; then the mixture was stirred overnight under reduced temperature, subjected to suction filtration, and the filter cake was dried under forced air for 8 hours at 50°C to afford 103.5g of a yellow solid powder with a yield of 89.27% and a liquid phase purity of 99.7%.

MS m/z: 555.2[M+H]⁺.

¹H NMR (400 MHz, DMSO-*d₆*) δ 9.81 (s, 1H), 8.82 (s, 1H), 8.46 (s, 2H), 8.21 (s, 1H), 8.04 (s, 1H), 7.62 (d, J = 8.2 Hz, 1H), 7.46 (d, J = 6.9 Hz, 1H), 7.34 (t, J = 7.7 Hz, 1H), 7.22 (s, 1H), 6.63 (dd, J = 17.0, 10.2 Hz, 1H), 6.29 (dd, J = 17.0, 1.9 Hz, 1H), 5.81 (dd, J = 10.2, 1.9 Hz, 1H), 4.96 (dd, J = 18.0 Hz, 2H), 3.94 (s, 3H), 3.72 (t, J = 6.3 Hz, 2H), 3.20 (t, J = 6.2 Hz, 2H), 2.92 (s, 3H), 2.67 (t, J = 5.4 Hz, 3H), 2.42 (s, 9H).

¹³C NMR (400 MHz, DMSO-*d₆*) δ167.18, 163.66, 153.15, 150.85, 143.77, 138.96, 138.23, 136.03, 131.17, 128.06, 127.39, 125.45, 125.30, 123.51, 122.65, 122.54, 122.40, 119.78, 116.57, 111.43, 111.23, 106.07, 62.51, 62.17, 61.83, 61.48, 47.45, 45.86, 39.73, 39.32, 33.68, 32.73

### Embodiment 3

### Synthesis process of compound III-1

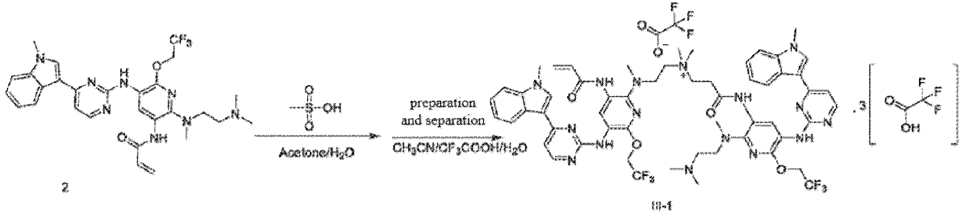

### Preparation of compound III-1

To a reaction flask were successively added a compound 2 (779.7g, 1.0eq), acetone (7.8L) and water (1.8L), followed by stirring and heating to 50-55°C, and a mixed solution of methanesulfonic acid (130.4g, 0.99eq) and acetone (2.3L) was added dropwise; after the dropwise addition was completed, stirring was performed under heat preservation for 48 hours; then, the reaction solution was concentrated under reduced pressure; after the concentration was completed, tetrahydrofuran (3.9L) and acetonitrile (3.9L) were added, beating under reflux and hot suction filtration were performed, and the mother solution was concentrated to afford 873g of a crude product. The crude product was subjected to liquid phase preparation to afford a compound III-1 with a purity of more than 99%.

### Liquid phase preparation method:

The above crude product was taken, dissolved with a mixed solvent of acetonitrile and N,N-dimethylformamide with a volume ratio of 1:1, prepared and separated with the following instrument and method, and frozen and dried to afford an impurity D.
Instrument: Shimadzu LC-20AP Prep HPLC (ACSSH PreL-GB)
Column: Luna C18 10µm, 250*50mm I.D.
Mobile phase A: a solution of 0.1% trifluoroacetic acid Mobile phase B: acetonitrile in water
Flow rate: 120mL/min Wavelength: 332nm
Gradient: mobile phase B 5-50%, 30min linear gradient Temperature: room temperature

MS m/z: 1137.6[M+H]⁺.

¹H NMR (400 MHz, DMSO-*d₆*) δ 9.92 (s, 1H), 9.72 (s, 1H), 8.62 (d, J = 9.9 Hz, 2H), 8.25 (t, J = 6.2 Hz, 3H), 8.11 (s, 1H), 7.99 (s, 1H), 7.55 (dd, J = 8.3, 2.8 Hz, 2H), 7.34 (t, J = 6.0 Hz, 2H), 7.28 (t, J = 7.6 Hz, 2H), 7.17 (dt, J = 14.4, 7.6 Hz, 2H), 6.24 (dd, J = 17.1, 1.9 Hz, 1H), 5.75 (dd, J = 10.2, 2.0 Hz, 1H), 5.01 - 4.90 (m, 4H), 3.90 (s, 6H), 3.73 (q, J = 7.6, 7.0 Hz, 4H), 3.59 (t, J = 7.0 Hz, 2H), 3.35 (q, J = 6.1 Hz, 2H), 3.15 (s, 6H), 3.04 (t, J = 7.7 Hz, 2H), 2.96 (d, J = 3.5 Hz, 6H), 2.86 (d, J = 4.1 Hz, 6H).

¹³C NMR (400 MHz, DMSO-*d₆*) δ167.79, 164.78, 163.86, 159.00, 158.66, 158.32, 157.99, 157.08, 150.49, 149.20, 137.91, 136.18, 134.93, 131.26, 128.13, 127.03, 125.49, 125.36, 122.81, 122.60, 125.25, 122.05, 121.84, 121.69, 120.80, 119.84, 117.86, 116.12, 115.90, 114.93, 111.99, 111.85, 110.84, 110.78, 106.4, 62.53, 62.19, 62.10, 61.76, 60.38, 60.10, 53.59, 50.16, 46.42, 45.25, 42.61, 39.30, 39.09, 33.34, 28.84

### Embodiment 4

### Synthesis process of compound VI-1

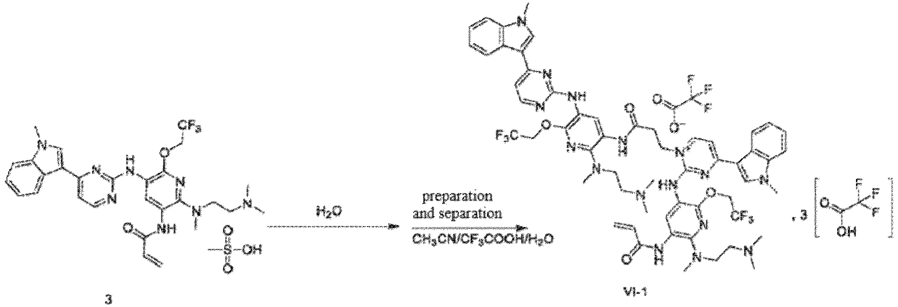

### Preparation of compound VI-1

To a reaction flask were successively added a compound 3 (800g) and water (8L), followed by stirring and heating to 80°C for reaction for 36 hours; then, the mixture was cooled to 20-30°C and subjected to suction filtration, and the filtrate was taken and concentrated under reduced pressure to afford 810g of a crude product.

To a reaction flask were successively added a compound 3 (500g) and water (5L), followed by stirring and heating to 80°C for reaction for 36 hours; then, the mixture was cooled to 20-30°C and subjected to suction filtration, and the filtrate was taken and concentrated under reduced pressure to afford 502g of a crude product.

The above 810g crude product and 502g crude product were combined and enriched by column chromatography to afford 462.6g of a preparative crude product sample. The preparative crude product sample was dissolved in a mixed solvent of N,N-dimethylformamide and acetonitrile with a volume ratio of 1:1.2, and was subsequently subjected to liquid phase preparation to afford a compound VI-1 with a purity of more than 99%.

Liquid phase preparation:
Instrument: Shimadzu 20A HPLC
Column: Ultimate LP-C18 4.6×150 mm, 5µm
Column temperature: 40°C
Flow rate: 1.5mL/min
Detector: UV 220nm, 215nm, 254nm
Mobile phase A: a solution of 0.1% trifluoroacetic acid in water
Mobile phase B: a solution of 0.1% trifluoroacetic acid in acetonitrile
Sample size: 1µL
Gradient:

| Time (min) | | A% | B% |
|---|---|---|---|
| | 0 | 90 | 10 |
| | 10 | 20 | 80 |
| | 15 | 20 | 80 |
| | 15.01 | 90 | 10 |
| | 20 | 90 | 10 |

MS m/z: 1137.5[M+H]⁺.

¹H NMR(400 MHz, DMSO-*d₆*) δ 10.31(s, 1H), 9.98(br, 1H), 9.93 (s, 2H), 9.84 (s, 1H), 8.67 (s, 1H), 8.50 (s, 1H), 8.37 (d, J = 7.1 Hz, 1H), 8.22 (t,) = 11.7 Hz, 2H), 7.56 (d, J = 8.3 Hz, 1H), 7.49 - 7.38 (m, 3H), 7.32 - 7.19 (m, 3H), 7.14 (t, J = 7.6 Hz, 1H), 6.99 (t, J = 7.6 Hz, 1H), 6.58 (dd, J = 17.0, 10.2 Hz, 1H), 6.29 (d, J = 17.0 Hz, 1H), 5.81 (d, J = 10.3 Hz, 1H), 4.96 (q, J = 9.0 Hz, 3H), 4.85 (q, J = 8.9 Hz, 3H), 4.60 (t, J = 6.4 Hz, 3H), 3.86 (d, J = 19.8 Hz, 8H), 3.69 (d, J = 6.9 Hz, 2H), 3.44 - 3.29 (m, 4H), 3.15 (t, J = 6.5 Hz, 2H), 3.02 (s, 3H), 2.91 - 2.83 (m, 15H).

¹³C NMR (400 MHz, DMSO-*d₆*) δ169.47, 163.71, 163.21, 161.94, 160.35, 158.50, 157.34, 150.92, 149.63, 148.23, 147.04, 146.83, 144.48, 137.66, 137.63, 135.25, 133.22, 131.85, 128.99, 128.66, 126.44, 125.50, 125.02, 123.18, 122.69, 122.47, 122.07, 121.77, 118.34, 116.13, 115.70, 112.44, 112.36, 110.37, 110.28, 107.02, 98.37, 61.42, 55.70, 55.67, 47.90, 44.48, 44.46, 44.19, 40.09, 39.00, 33.47, 33.15, 33.01

### Embodiment 5

### Synthesis process of compound V-1

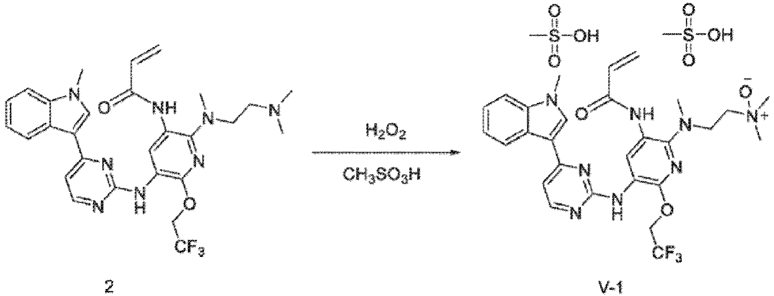

### Preparation of compound V-1

A compound 2 (20g, 35mmol, 1.0eq) and ethanol (200mL) were added to a reaction flask, followed by stirring and addition of 16g of hydrogen peroxide (140mmol, 4.0eq), the mixture was heated to 50°C and stirred overnight under heat preservation for reaction. After the reaction was monitored to be completed, methyl tert-butyl ether (500mL) was added dropwise; after the dropwise addition was completed, the reaction system was cooled to room temperature to precipitate a large amount of solids, and suction filtration was performed to afford a free wet product.

The above wet product was transferred into the flask, a mixed solvent (240mL, 15:1) of acetone and water was added and stirred evenly, the temperature was controlled at 20-30°C; and a mixed solution of 13.5g of methanesulfonic acid (140mmol, 4eq) and acetone (60mL) was added dropwise. After the dropwise addition was completed, stirring was continued for 4 hours, and a reaction solution was subjected to suction filtration. The solid obtained by the suction filtration was transferred into the reaction flask, and acetonitrile (300mL) was added; stirring is continued for 3 hours at 20-30°C, and after stirring, methyl tert-butyl ether (300mL) was added dropwise; after the dropwise addition was completed, stirring was continued for 1 hour, and the reaction solution was subjected to suction filtration; the filter cake was dried at 50°C to afford 24.0g of a yellow solid with a purity of 99.9% and a yield of 87.8%.

MS m/z: 585.4[M+H]⁺.

¹H NMR (400 MHz, DMSO-d6) δ 12.38 (s, 1H), 9.72 (s, 1H), 8.77 (s, 1H), 8.20 (br, 1H), 7.92 (br, 1H), 7.61 (d, J = 8.3 Hz, 1H), 7.43 (d, J = 6.8 Hz, 1H), 7.33 (t, J = 7.5 Hz, 1H), 7.23 (br, 1H), 6.55 (dd, J = 17.0, 10.3 Hz, 1H), 6.23 (dd, J = 17.1, 2.0 Hz, 1H), 5.75 (dd, J = 10.1, 2.0 Hz, 1H), 4.95 (q, J = 9.0 Hz, 2H), 3.93 (s, 3H) 3.91 (s, 4H), 3.53 (s, 6H), 3.01 (s, 3H), 2.41 (s, 6H).

¹³C NMR (126 MHz, DMSO-d6) δ 167.08, 163.96, 153.29, 151.22, 138.85, 138.20, 131.25, 127.04, 125.46, 125.06, 123.48, 122.85, 122.68, 115.10, 111.44, 111.20, 106.02, 64.51, 62.21, 61.94, 61.67, 56.07, 45.34, 39.71, 38.70, 33.66

### Test embodiment 1: proliferation inhibition effect on PC-9-EGFR-Del19-T790M-C797S cells

The *in vitro* proliferation inhibition activity of the compound on PC-9-EGFR-Del19-T790M-C797Slcells where Del19/T790M/C797S triple mutation exists in exogenously stably overexpressed EGFR protein of human non-small cell lung cancer PC-9 was determined by sulforhodamine B method (SRB method).

Cell source: PC-9-EGFR-Del19-T790M-C797S cells were purchased from Nanjing Cobioer Biosciences Co., Ltd.

PC-9-EGFR-Del19-T790M-C797S cells were cultured in an RPMI1640 complete medium containing puromycin (2µg/ml) and 10% fetal bovine serum. PC-9-EGFR-Del19-T790M-C797S cells in a logarithmic growth phase were taken, seeded in a 96-well plate at a cell density of 5000 cells/135µL complete culture medium/well, placed in a thermostatic incubator containing 5% CO₂ at 37°C and cultured for 24 hours to ensure complete anchorage of cells. Each compound was dissolved in dimethyl sulfoxide (DMSO) in advance to prepare a 10mM stock solution, and then the compound was successively diluted with DMSO and the complete culture medium. The 96-well plates in which the cells were seeded were taken out, and one of the plates was used as a growth-free control group (a medium control group without cell growth at 0 hour); 15µL of compounds with different concentrations were added to each well of other 96-well plates to achieve the final concentrations of 2500, 625, 156.25, 39.06, 9.77, 2.44, 0.61, 0.15, 0.04, and 0.01nM, and three duplicate wells were set for each compound concentration, a negative control group (a medium control group which contained cells but had no compound added) was provided, and the concentration of DMSO in each well was 0.5%.

The culture of the reserved growth-free control group was terminated immediately, and other 96-well plates were continued to be cultured for 72 hours in the thermostatic incubator containing 5% CO₂ at 37°C before culture termination. The method for culture termination was as follows: each well was added with 50µL of pre-cooled (4°C) 50% trichloroacetic acid aqueous solution, placed and fixed for 1 hour at 4°C, washed with purified water for at least 5 times, and dried naturally in the air or dried in an oven at 60°C.

A 4mg/mL SRB solution was prepared by purified water containing 1% glacial acetic acid; 100µL was added in each well, stained for 1 hour at room temperature, with the supernatant discarded, washed for at least 5 times with 1% glacial acetic acid to remove non-specific binding, and dried for future use. Each well was added with 150µL of a 10mM tris-hydroxymethylaminomethane hydrochloride solution (a Tris-HCl solution), an optical density value (OD value) at a wavelength of 510nm was measured, and data was processed to calculate a cell proliferation inhibition rate. Cell proliferation inhibition rate = [(ODnegative control group at 72h-ODcompound administration group at 72h)/(ODnegative control group at 72h-ODgrowth-free control group)]×100%.

GraphPad Prism 8.3 software was used for data analysis, and non-linear S-curve regression was used for fitting the data to obtain a dose-effect curve, from which an IC₅₀ value was calculated, and the results are shown in Table 1.

**Table 1**

| **Compound** | **PC-9-EGFR-Del19-T790M-C797S IC₅₀ (µM)** |
|---|---|
| Compound I | 2.932 |
| Compound II | 0.173 |
| Compound III | 0.147 |
| Compound VI | 0.214 |
| Compound V | 0.564 |

The test results show that the compound of the present invention has good proliferation inhibition activity on PC-9-EGFR-Del19-T790M-C797S cells.

## Claims

1. A compound as represented by formula I or a pharmaceutically acceptable salt thereof, 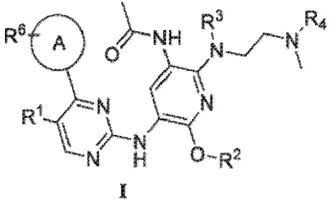
wherein ring A is 5- to 10-membered heteroaryl;
R¹ is H, halogen, C₁-C₄ alkyl, or C₁-C₄ alkyl substituted with halogen;
R² is C₁-C₄ alkyl or C₁-C₄ alkyl substituted with halogen;
R³ is H, C₁-C₄ alkyl, or C₁-C₄ alkyl substituted with halogen;
R⁴ is H, C₁-C₄ alkyl, or C₁-C₄ alkyl substituted with halogen; and
R⁶ is H, halogen, -CN, C₁-C₄ alkyl, or C₁-C₄ alkyl substituted with halogen.

2. The compound as represented by formula I of claim 1 or a pharmaceutically acceptable salt thereof, which satisfies one or more of the following conditions:
(1) the halogen is independently F, Cl, Br, or I;
(2) the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl;
(3) the C₁-C₄ alkyl substituted with halogen is independently trifluoromethyl, -CH₂CH₂Cl, or -CH₂CF₃;
(4) the ring A is indolyl, indazolyl, benzothiazolyl, pyrimidinyl, or pyrazolyl;
(5) the pharmaceutically acceptable salt of the compound as represented by formula I is a mesylate salt of the compound as represented by formula I;
(6) the compound as represented by formula I can be 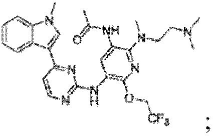 and
(7) the pharmaceutically acceptable salt of the compound as represented by formula I can be

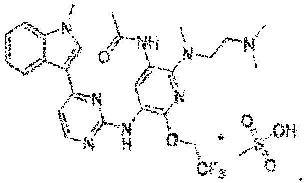

3. A compound as represented by formula II,

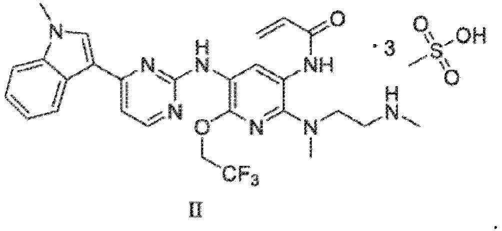

4. A compound as represented by formula III or a pharmaceutically acceptable salt thereof, 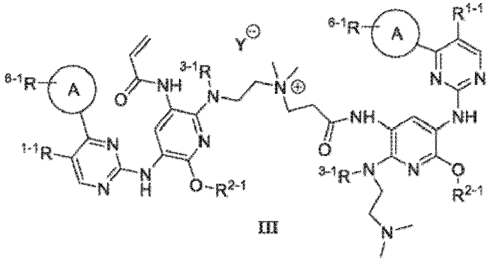
wherein ring A is 5- to 10-membered heteroaryl;
R¹⁻¹ is H, halogen, C₁-C₄ alkyl, or C₁-C₄ alkyl substituted with halogen;
R²⁻¹ is C₁-C₄ alkyl or C₁-C₄ alkyl substituted with halogen;
R³⁻¹ is H, C₁-C₄ alkyl, or C₁-C₄ alkyl substituted with halogen;
R⁶⁻¹ is H, halogen, -CN, C₁-C₄ alkyl, or C₁-C₄ alkyl substituted with halogen; and
Y^{Θ} is trifluoroacetate radical or mesylate radical.

5. The compound as represented by formula III of claim 4 or a pharmaceutically acceptable salt thereof, which satisfies one or more of the following conditions:
(1) the halogen is independently F, Cl, Br, or I;
(2) the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl;
(3) the C₁-C₄ alkyl substituted with halogen is independently trifluoromethyl, -CH₂CH₂Cl, or -CH₂CF₃;
(4) the ring A is indolyl, indazolyl, benzothiazolyl, pyrimidinyl, or pyrazolyl;
(5) the pharmaceutically acceptable salt of the compound as represented by formula III is a trifluoroacetate salt of the compound as represented by formula III;
(6) the compound as represented by formula III can be 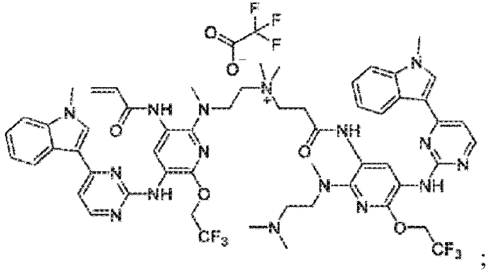 and
(7) the pharmaceutically acceptable salt of the compound as represented by formula III can be

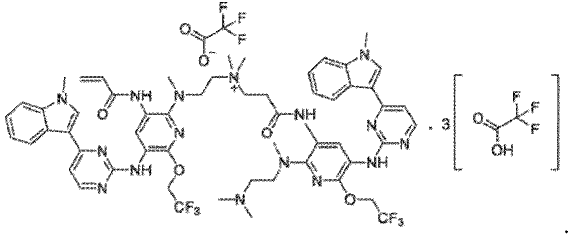

6. A compound as represented by formula VI or a pharmaceutically acceptable salt thereof, 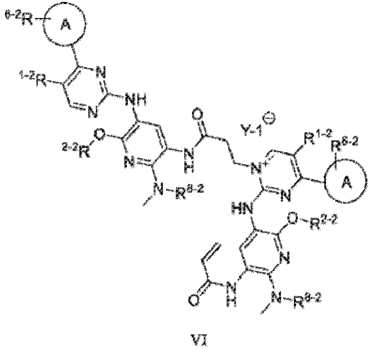
wherein ring A is 5- to 10-membered heteroaryl;
R¹⁻² is H, halogen, C₁-C₄ alkyl, or C₁-C₄ alkyl substituted with halogen;
R²⁻² is C₁-C₄ alkyl or C₁-C₄ alkyl substituted with halogen;
R⁶⁻² is H, halogen, -CN, C₁-C₄ alkyl, or C₁-C₄ alkyl substituted with halogen;
R⁸⁻² is C₁-C₄ alkyl or C₁-C₄ alkyl substituted with 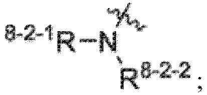
R⁸⁻²⁻¹ and R⁸⁻²⁻² are independently hydrogen, C₁-C₄ alkyl, or C₁-C₄ alkyl substituted with halogen; and
Y-1^{Θ} is trifluoroacetate radical or mesylate radical.

7. The compound as represented by formula VI of claim 6 or a pharmaceutically acceptable salt thereof, which satisfies one or more of the following conditions:
(1) the halogen is independently F, Cl, Br, or I;
(2) the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl;
(3) the C₁-C₄ alkyl substituted with halogen is independently trifluoromethyl, -CH₂CH₂Cl, or -CH₂CF₃;
(4) the ring A is indolyl, indazolyl, benzothiazolyl, pyrimidinyl, or pyrazolyl;
(5) the pharmaceutically acceptable salt of the compound as represented by formula VI is a trifluoroacetate salt of the compound as represented by formula VI;
(6) the compound as represented by formula VI can be 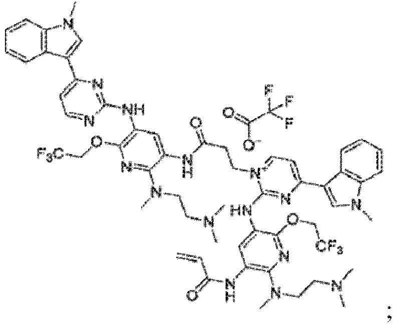 and
(7) the pharmaceutically acceptable salt of the compound as represented by formula VI can be the following compound,

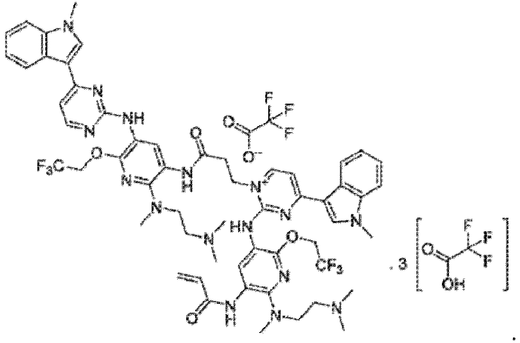

8. A compound as represented by formula V or a pharmaceutically acceptable salt thereof, 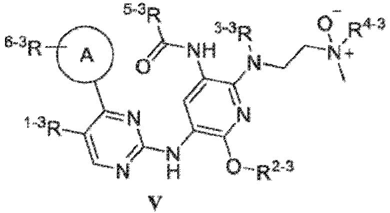
wherein ring A is 5- to 10-membered heteroaryl;
R¹⁻³ is H, halogen, C₁-C₄ alkyl, or C₁-C₄ alkyl substituted with halogen;
R²⁻³ is C₁-C₄ alkyl or C₁-C₄ alkyl substituted with halogen;
R³⁻³ is H, C₁-C₄ alkyl, or C₁-C₄ alkyl substituted with halogen;
R⁴⁻³ is H, C₁-C₄ alkyl, or C₁-C₄ alkyl substituted with halogen;
R⁵⁻³ is 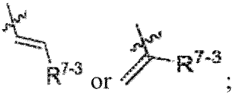
R⁶⁻³ is H, halogen, -CN, C₁-C₄ alkyl, or C₁-C₄ alkyl substituted with halogen; and
R⁷⁻³ is hydrogen, C₁-C₄ alkyl, or C₁-C₄ alkyl substituted with halogen.

9. The compound as represented by formula V of claim 8 or a pharmaceutically acceptable salt thereof, which satisfies one or more of the following conditions:
(1) the halogen is independently F, Cl, Br, or I;
(2) the C₁-C₄ alkyl and C₁-C₄ alkyl in the C₁-C₄ alkyl substituted with halogen are independently methyl, ethyl, n-propyl, isopropyl, n-butyl, isobutyl, sec-butyl, or tert-butyl;
(3) the C₁-C₄ alkyl substituted with halogen is independently trifluoromethyl, -CH₂CH₂Cl, or -CH₂CF₃;
(4) the ring A is indolyl, indazolyl, benzothiazolyl, pyrimidinyl, or pyrazolyl;
(5) the pharmaceutically acceptable salt of the compound as represented by formula V is a mesylate salt of the compound as represented by formula V;
(6) the compound as represented by formula V can be 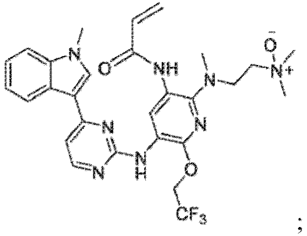 and
(7) the pharmaceutically acceptable salt of the compound as represented by formula V can be

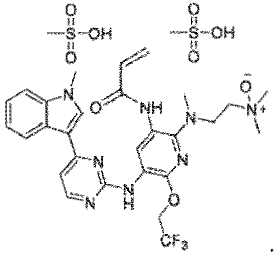

10. A preparation method for a compound as represented by formula I-a, comprising the step of: salt-forming reaction of the compound as represented by formula I with methanesulfonic acid in a ketone solvent to afford a compound as represented by formula I-a; 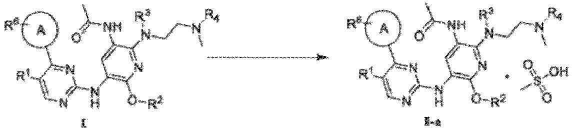
wherein each group is as defined in any one of claim 1 or 2; and
wherein the ketone solvent can be acetone;
the salt-forming reaction can be at a temperature of 40-60°C, for example, 50°C; and
the preparation method for the compound as represented by formula I-a can further comprise the step of: amide reaction of a compound M with acetyl chloride in an organic solvent under the action of a base to afford the compound as represented by formula I; 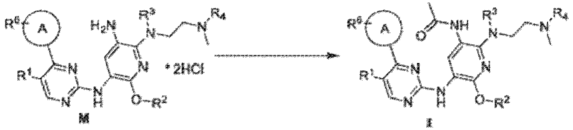
wherein the organic solvent can be dichloromethane;
the base can be triethylamine; and
the amide reaction can be at a temperature of -10°C-0°C.

11. A preparation method for a compound as represented by formula I, comprising the step of: amide reaction of a compound M with acetyl chloride in an organic solvent under the action of a base to afford a compound as represented by formula I; 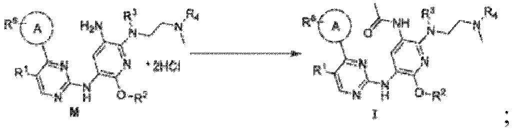 wherein each group is as defined in any one of claim 1 or 2; and the amide reaction can be under the reaction conditions as defined in claim 10.

12. A preparation method for a compound as represented by formula II, comprising the step of: reaction of a compound P with methanesulfonic acid in a ketone solvent to afford a compound as represented by formula II; 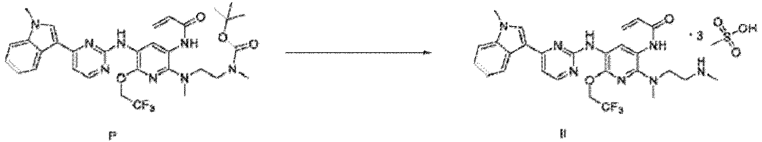
wherein the ketone solvent can be acetone;
the reaction can be at a temperature of 30-50°C, for example, 35°C;
the reaction can be for 2-6 hours, preferably 4 hours; and
the reaction can be subject to post-treatment by beating with ethyl acetate and drying at 50°C; and
the preparation method for the compound as represented by formula II can further comprise:
(1) amination reaction of a compound P-4 with N,N'-dimethylethylenediamine in N,N-dimethylformamide to afford a compound P-3;
(2) reaction of the compound P-3 with di-tert-butyl dicarbonate in dichloromethane in the presence of triethylamine to afford a compound P-2;
(3) reduction of the compound P-2 with sodium hydrosulfite in ethanol and water to afford a compound P-1; and
(4) reaction of the compound P-1 with 3-chloropropionyl chloride in dichloromethane in the presence of triethylamine to afford the compound P;

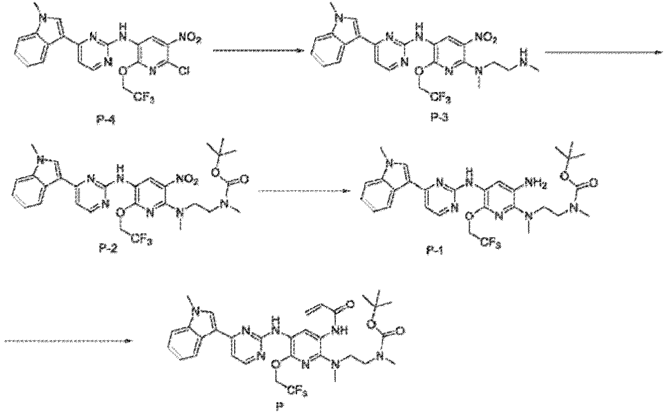

13. A preparation method for a compound as represented by formula III or a pharmaceutically acceptable salt thereof, comprising the steps of reaction of an acid with a compound N in a solvent, and optionally purification by high performance liquid chromatography to afford the compound as represented by formula III or a pharmaceutically acceptable salt thereof; 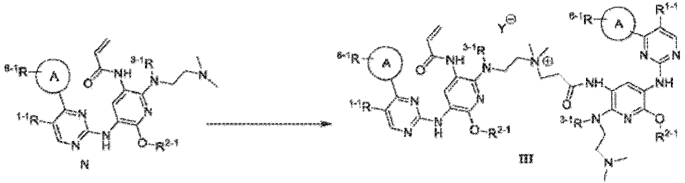
wherein each group is as defined in any one of claim 4 or 5; and
wherein the solvent is selected from one or a mixture of acetone and water;
the acid can be methanesulfonic acid and trifluoroacetic acid;
the reaction can be at a temperature of 50-55°C;
the reaction can be for 36-72 hours, preferably 48 hours; and
the purification by high performance liquid chromatography can be under the following conditions: mobile phase A: a solution of 0.1% trifluoroacetic acid in water; mobile phase B: acetonitrile; flow rate: 120mL/min; and wavelength: 332nm.

14. A preparation method for a compound as represented by formula VI or a pharmaceutically acceptable salt thereof, comprising the steps of: addition reaction of a compound N-1 by heating to 70-90°C in a solvent, and optionally purification by high performance liquid chromatography to afford the compound as represented by formula VI or a pharmaceutically acceptable salt thereof; 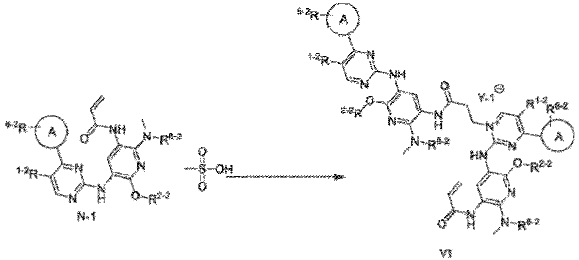
wherein each group is as defined in any one of claim 6 or 7; and
wherein the solvent can be water;
the addition reaction can be at a temperature of 70-90°C, preferably 80°C; and
the purification by high performance liquid chromatography can be under the following conditions: mobile phase A: a solution of 0.1% trifluoroacetic acid in water; mobile phase B: a solution of 0.1% trifluoroacetic acid in acetonitrile; flow rate: 1.5 mL/min.

15. A preparation method for a compound as represented by formula V or a pharmaceutically acceptable salt thereof, comprising the steps of: oxidation reaction of a compound N-2 in an organic solvent in the presence of an oxidizing agent H₂O₂, and further optionally salt-forming reaction, to afford the compound as represented by formula V or a pharmaceutically acceptable salt thereof; 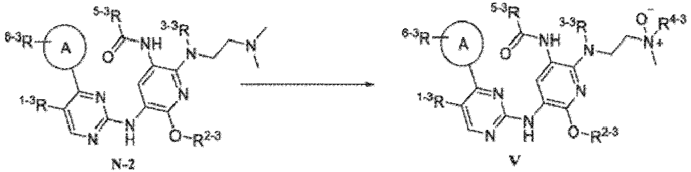
wherein each group is as defined in any one of claim 8 or 9; and
wherein the organic solvent can be ethanol;
the oxidation reaction can be at a temperature of 40-60°C, preferably 50°C; and
the salt-forming reaction can be under the following conditions: directly salt-forming by an oxidation product of the compound N-2 with methanesulfonic acid in a mixed solvent of acetone and water at a temperature of 20-30°C.

16. A pharmaceutical composition, comprising a therapeutically effective amount of a substance X and pharmaceutically acceptable supplementary materials;
wherein the substance X is any one of the following substances:
(1) the compound as represented by formula I of claim 1 or a pharmaceutically acceptable salt thereof;
(2) the compound as represented by formula II of claim 3;
(3) the compound as represented by formula III of claim 4 or a pharmaceutically acceptable salt thereof;
(4) the compound as represented by formula VI of claim 6 or a pharmaceutically acceptable salt thereof;
or (5) the compound as represented by formula V of claim 8 or a pharmaceutically acceptable salt thereof.

17. Use of a substance X or the pharmaceutical composition of claim 16 for preparation of an EGFR inhibitor;
wherein the EGFR inhibitor is an inhibitor of EGFR Del19/T790M/C797S mutation; and
the substance X is any one of the following substances:
(1) the compound as represented by formula I of claim 1 or a pharmaceutically acceptable salt thereof;
(2) the compound as represented by formula II of claim 3;
(3) the compound as represented by formula III of claim 4 or a pharmaceutically acceptable salt thereof;
(4) the compound as represented by formula VI of claim 6 or a pharmaceutically acceptable salt thereof;
or (5) the compound as represented by formula V of claim 8 or a pharmaceutically acceptable salt thereof.

18. Use of a substance X or the pharmaceutical composition of claim 16 in preparation of a medicament for treatment and/or prevention of a disease mediated by EGFR or for treatment and/or prevention of a cancer;
the disease mediated by EGFR is a disease mediated by EGFR Del19/T790M/C797S mutation;
the substance X is any one of the following substances:
(1) the compound as represented by formula I of claim 1 or a pharmaceutically acceptable salt thereof;
(2) the compound as represented by formula II of claim 3;
(3) the compound as represented by formula III of claim 4 or a pharmaceutically acceptable salt thereof;
(4) the compound as represented by formula VI of claim 6 or a pharmaceutically acceptable salt thereof;
or (5) the compound as represented by formula V of claim 8 or a pharmaceutically acceptable salt thereof.
